# EUROPEAN PATENT SPECIFICATION

(11) **EP 4 197 480 B1**
(45) Date of publication and mention of the grant of the patent: **31.12.2025**
(21) Application number: 21917208.7
(22) Date of filing: 24.11.2021
(51) Int. Cl.: A61B 34/30, B25J 9/00, B25J 9/10, B25J 18/00

(54) **CONNECTING ARM, ROBOTIC ARM, AND ROBOT**
VERBINDUNGSARM, ROBOTERARM UND ROBOTER
BRAS DE LIAISON, BRAS ROBOTIQUE ET ROBOT

(30) Priority: 06.01.2021 CN 202110013227
(43) Date of publication of application: 21.06.2023
(73) Proprietor: Cornerstone Technology (Shenzhen) Limited, Shenzhen, Guandong 518066 (CN)
(72) Inventor: WANG, Zerui, Shenzhen, Guangdong 518066 (CN)
(74) Representative: Dennemeyer & Associates S.A.
(86) International application number: PCT/CN2021/132877
(87) International publication number: WO 2022/148167

(56) References cited:
- WO-A1-00/41855
- WO-A1-2015/103089
- WO-A1-97/03395
- WO-A2-02/34477
- CN-A- 102 990 675
- CN-A- 107 498 581
- CN-A- 110 948 476
- CN-A- 110 948 476
- CN-A- 112 754 663
- CN-U- 207 534 845
- CN-U- 207 534 845
- CN-U- 208 481 467
- CN-U- 209 408 533
- CN-U- 209 408 533
- CN-U- 210 931 809
- US-A1- 2010 224 023
- US-B1- 6 457 380

## Description

### TECHNICAL FIELD

The present disclosure relates to the technical field of machinery, in particular to a link, a robotic arm and a robot.

### BACKGROUND

Medical surgical micro-instruments are widely used in various operations due to the advantages of accurate positioning, stable operation, desirable flexibility, large working range, radiation and infection protection, and the like. The surgical robot is a type of medical equipment. In the operation, the patient on the patient's bed is pushed to a position in front of the surgical robot, and the surgical robot has its multiple robotic arms controlled to move onto the patient's body to perform the corresponding surgery via one or more preset hole of the patient's skin. During the operation, a surgical micro-instrument held by one of the robotic arms can be rotated around a respective rotation point (called RCM, Remote Center of Motion, point) at the respective preset hole and inserted into the patient via the respective preset hole.

The existing surgical robots have been developed to have a function of realizing the rotation of the robotic arm around the RCM point by the constraint of a mechanical parallelogram mechanism. For example, steel wires or straps are employed for transmission and constraint of the rotation of the links of the robotic arm, so that a rod of an effector unit connected to an end of the robotic arm exhibits a coupling characteristic of a parallelogram. In a structure of the robotic arm in which multiple links are connected in series and which is very long, the compactness and high rigidity of the robotic arm with a strap drive train have a crucial impact on the surgical performance of the surgical robot.

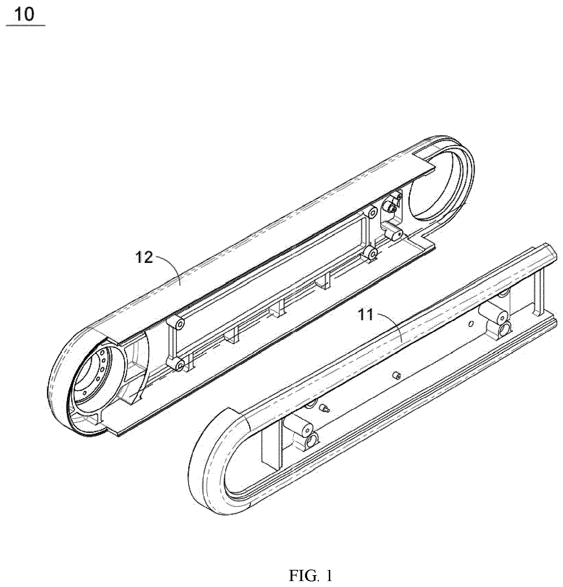

FIG. 1 shows an existing structure employed for a link of a robotic arm, the link includes an upper cover 11 and a lower bottom plate 12 that are connected by a fastener, an accommodating cavity is formed between the upper cover 11 and the lower bottom plate 12 to receive a flexible transmission assembly. The link of this structure requires higher rigidity of the lower bottom plate 12, especially the link at the distal end of the robotic arm which is subjected to both a large bending moment and a large torque during its movement and the operation of the medical staff. Since there is a seam at the connection between the upper cover 11 and the lower bottom plate 12, a displacement between layers occurs at the seam, and meanwhile the lower bottom plate 12 is subjected to a relatively large torsional deformation. For the elongated robotic arm including multiple links connected in series, the torsional deformations of the links accumulate to result in a large operating error of the end effector, thereby reducing the accuracy of the surgical robot.

In addition, the structure of the link also brings some difficulties to the maintenance of the flexible transmission assembly. In general, a flexible member of the flexible transmission assembly is screwed on an arc surface of a pulley by means of a bolt and the like, and a tensioning means for the flexible member is correspondingly disposed on the arc surface of the pulley. When the flexible member needs to be tensioned, the structure of the upper cover 11 and the lower bottom plate 12 prevent, to a certain extent, access of a screwdriver or a wrench to the bolt. In this case, an additional operation opening on the lower bottom plate 12 is required for the tension, measurement and maintenance of the pulley and steel strap.

CN 110 948 476 A discloses a horizontal multi-joint robot including a base, a large arm component, a small arm component, and an action shaft. The base includes a base portion in which a driving unit is arranged and an arm cylinder in which a transmission unit is arranged. The large arm component is connected with the base portion through the arm cylinder and can rotate around an axis J1. A driving arm is arranged between the large arm component and the arm cylinder and rotates around the axis J1. A front end of the driving arm is connected with an attached rod, and the other end of the attached rod is connected with the small arm component. The small arm component rotates around an axis J2 under the driving of a planar four-link mechanism. The action shaft is arranged at the other end of the small arm component and can rotate around an axis J3 and move up and down.

WO 97/03395 A1 discloses a robot including a shoulder motor directly driving a shoulder joint, an elbow motor directly driving an elbow joint, a first end effector drive pulley driven by an elbow motor, and a second end effector drive pulley coupled to the first end effector drive pulley.

CN 209 408 533 U discloses a horizontal multi-joint industrial robot. The manipulator includes a base, a large arm component, a small arm component, and an action shaft. One end of the large arm component is connected with the base and can rotate around an axis J1, and one end of the small arm component is mounted at the other end of the large arm component and can rotate around an axis J2. The action shaft is arranged at the other end of the small arm component and can do lifting motion and rotating motion relative to the small arm component. An end effector for operating respective components is arranged at an end of the action shaft. The large arm component and the small arm component are connected through a connecting cylinder for increasing the distance between the large arm component and the small arm component. The connecting cylinder and the small arm component synchronously rotate around the axis J2.

CN 207 534 845 U discloses a link arm of a mechanical manipulator which includes a main arm, a first joint flange, and a second joint flange. A main cavity is formed in the main arm which is provided with a side opening. The main arm includes a motor mounting seat, a sprocket mounting base, two synchronous belt wheel mounting bases, and two synchronous belt tensioner mounting bases.

WO 00/41855 A1 discloses a wafer handling robot for transporting workpieces. The robot includes a base comprising a rigid backbone for providing stability to the robot. The base further includes a mast, a linear drive system for translating the mast, and a shoulder drive system for rotating the mast. The shoulder drive system includes a harmonic drive reduction system for providing output rotation of the mast for rotation with the mast, and a distal link rotatably mounted to the proximal link. An end effector for supporting workpieces is rotationally mounted to the distal end of the distal link. An elbow drive is mounted to the proximal link, extending down into the mast section, for driving rotation of the distal link with respect to the proximal link.

WO 2015/103089 A1 discloses a substrate-transporting robot apparatus. The robot apparatus may include an upper arm, a forearm independently rotatable relative to the upper arm, a wrist member independently rotatable relative to the forearm, and an end effector adapted to carry a substrate. In some aspects, the independent rotation is provided by a robot drive assembly having a second driving pulley mounted for rotation on a first driving pulley.

WO 02/34477 A2 discloses a robot drive assembly for moving a working tool in x, y, z and theta directions including three independent, coaxially nested tubes, each tube being driven around a common central axis by drive belts attached to separate drive motors located in a mounting flange associated with the outermost tube. The motors, and the tubes which they drive, provide horizontal rotary motion to a robot arm attached to the upper end of the outer tube and the wrist and elbow of that arm. A fourth motor controls vertical motion of the whole assembly. The robot system also includes motor position adjustment structure and belt tension structure designed for ease of use and to eliminate movement of tensioned components once locked in position.

US 2010/0224023 A1 discloses a support arm used in an industrial robot includes a first joint portion, a second joint portion, and a connecting portion between the first and second joint portions. The connecting portion includes a plurality of connecting walls. The plurality of connecting walls and the first and second joint portions cooperatively define a cavity. One of the connecting walls defines an opening communicating with the cavity and forms a plurality of reinforced ribs extending from the periphery of the opening towards the cavity of the support arm.

To this end, the present disclosure provides a link, a robotic arm and a robot to at least partially solve the problems in the existing knowledge.

### SUMMARY

A series of concepts in simplified form have been introduced in the summary section, which are described in further detail in the detailed description section. The summary section of the present disclosure does not mean to attempt to limit the key features and essential technical features of the claimed technical solution, nor does it mean to attempt to determine the protection scope of the claimed technical solution. The invention is defined by the independent claim. The dependent claims define advantageous embodiments.

To at least partially solve the aforementioned technical problems, according to the invention, a robotic arm is disclosed, the robotic arm including at least one link having a length and defining an inner cavity extending in a length direction of the link, wherein at least a part of the inner cavity is seamlessly enclosed in section perpendicular to the length direction.

Since at least a part of the inner cavity is seamlessly enclosed in section perpendicular to the length direction of the link, the smooth transmission of the force flow and the continuity of the force can be ensured, and the link having a relative small wall thickness and a relative simple structure can meet the requirements for rigidity and torsional strength.

According to the invention, the link is one-piece.

According to the invention, the link includes at least one recess at an outer surface of the link.

According to the invention, the recess is configured to accommodate at least one of wires, circuit boards, sensors, connecting flanges and other mechanical and electrical parts of the robot arm.

According to the invention, the inner cavity is in communication with two end openings defined at both ends of the link in the length direction respectively.

According to the present solution, the flexible transmission assembly can enter into the inner cavity through the end opening, and the end opening is configured to provide an access for a wrench to perform maintenance at the end such as tightening, thereby facilitating the mounting and tensioning of the flexible member on the pulleys and the maintenance of the transmission element such as the pulleys.

According to the invention, the inner cavity is further in communication with two pairs of side openings, wherein a pair of side openings is defined at an end of the link in the length direction and is oppositely arranged on both sides of the link respectively, and the other pair of side openings is defined at the other end of the link in the length direction and is oppositely arranged on both sides of the link respectively.

According to the present solution, the side opening is configured to provide an access for a wrench to perform maintenance at the side such as tightening, thereby facilitating the operation and maintenance of the transmission elements such as the pulleys.

According to the invention, the robotic arm includes at least two links, which are connected end to end and adjacent two of which are pivotably connected.

According to the invention, the robotic arm further includes at least two flexible transmission assemblies and at least two pivot shafts, in the inner cavity of each of the links is disposed at least one of the flexible transmission assemblies, and the flexible transmission assemblies disposed in the respective inner cavities of the adjacent two of the links are connected by a respective one of the pivot shafts.

Optionally, in the inner cavity of each of the links is disposed one flexible transmission assembly which comprises a pair of pulleys and a flexible member tensioned on the pair of pulleys, the pair of pulleys are respectively disposed at the side openings at both ends of the inner cavity and enter into the inner cavity through the end openings, and the respective one of the pivot shafts is connected with the pulleys in the respective inner cavities of the adjacent two of the links through the side openings.

According to the invention, the robotic arm further includes an end cover for each of the end openings and a side cover for each of the side openings, and the side cover is disposed at a respective one of the side openings and connected with the link.

According to the invention, a link defining an inner cavity is disclosed, wherein the inner cavity extends in a length direction of the link from end to end of the link, and the link is integrally formed.

According to the link of the present disclosure, since the link is one-piece, the smooth transmission of the force flow and the continuity of the force can be ensured, and the link having a relative small wall thickness and a relative simple structure can meet the requirements for rigidity and torsional strength.

Optionally, the inner cavity extends straight from end to end of the link.

Optionally, a cross section of the inner cavity has a shape of a rectangle, a circle or an ellipse.

According to the invention, the link includes at least one recess at an outer surface of the link.

According to the invention, the inner cavity is in communication two end openings defined at both ends of the link in the length direction respectively, and each of the two end openings has a shape and a size which are adapted to a shape and a size of the cross section of the inner cavity.

According to the invention, the link includes a main body and a supporting portion integrally formed with the main body, the inner cavity is defined by the main body, and the supporting portion is configured to support a flexible transmission assembly and/or limit a position of the flexible transmission assembly.

According to a third aspect of the present disclosure, a robotic arm, which includes at least one link according to any one of the above second aspect, is disclosed.

According to the invention, the robotic arm includes at least two links which are connected end to end and adjacent two of which are pivotably connected.

According to the invention, the robotic arm further includes at least two flexible transmission assemblies and at least two pivot shafts, in the inner cavity of each of the links is disposed at least one of the flexible transmission assemblies, and the flexible transmission assemblies disposed in the respective inner cavities of the adjacent two of the links are connected by a respective one of the pivot shafts.

Optionally, in the inner cavity of the each of the links is disposed one flexible transmission assembly which comprises a pair of pulleys and a flexible member tensioned on the pair of pulleys, and the respective one of the pivot shafts is connected with the pulleys in the respective inner cavities of the adjacent two of the links.

According to a fourth aspect of the present disclosure, a robot, which includes at least one robotic arm according to any one of the above first and third aspects, is disclosed.

According to the robot of the present disclosure, since at least a part of the inner cavity is seamlessly enclosed in section perpendicular to the length direction of the link and/or the link is one-piece, the smooth transmission of the force flow and the continuity of the force can be ensured, and the link having a relative small wall thickness and a relative simple structure can meet the requirements for rigidity and torsional strength.

### BRIEF DESCRIPTION OF THE DRAWINGS

The following drawings of the present disclosure are incorporated herein as a part of the present disclosure for understanding of the present disclosure. The drawings illustrate embodiments and description of the present disclosure, which is used to explain the principle of the present disclosure.
FIG. 1 is a perspective exploded schematic view of a link of a robotic arm of a robot in the existing knowledge;
FIG. 2 is a schematic perspective view of a partial structure of a robot according to a preferred embodiment of the present disclosure, in which a robotic arm and an end effector are shown;
FIG. 3 is a schematic perspective view of a partial structure of the robot in FIG. 2, in which a robotic arm and an end effector are shown and a partial structure of the robotic arm is shown in an exploded view;
FIG. 4 is a perspective view of a link of the robotic arm in FIG. 3;
FIG. 5 is another perspective schematic view of the link of the robotic arm in Fig. 3; and
FIG. 6 is a schematic cross-sectional view of the robotic arm in FIG. 5.

### DETAILED DESCRIPTION

In the following description, numerous specific details are set forth in order to understand the present disclosure thoroughly. It will be apparent, however, for those skilled in the art that embodiments of the present disclosure may be practiced without one or more of these details. In other examples, some technical features well-known in the art are not described in order to avoid confusion with the embodiments of the present disclosure.

For a thorough understanding of the embodiments of the present disclosure, detailed structures will be presented in the following description. Obviously, the implementation of the embodiments of the present disclosure is not limited to the specific details familiar to those skilled in the art. It should be noted that the ordinal numbers such as "first" and "second" quoted in the present disclosure are merely identifications, and do not have any other meanings, such as a specific order. Also, for example, the term "a first element" does not by itself imply the presence of "a second element", nor does the term "a second element" by itself imply the presence of "a first element". The terms "upper", "lower", "front", "rear", "left", "right" and similar expressions used in the present disclosure are for the purpose of illustration and not limitation.

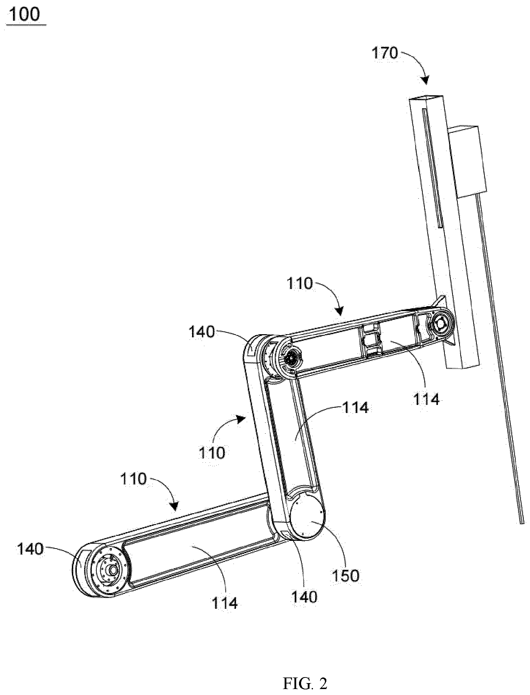

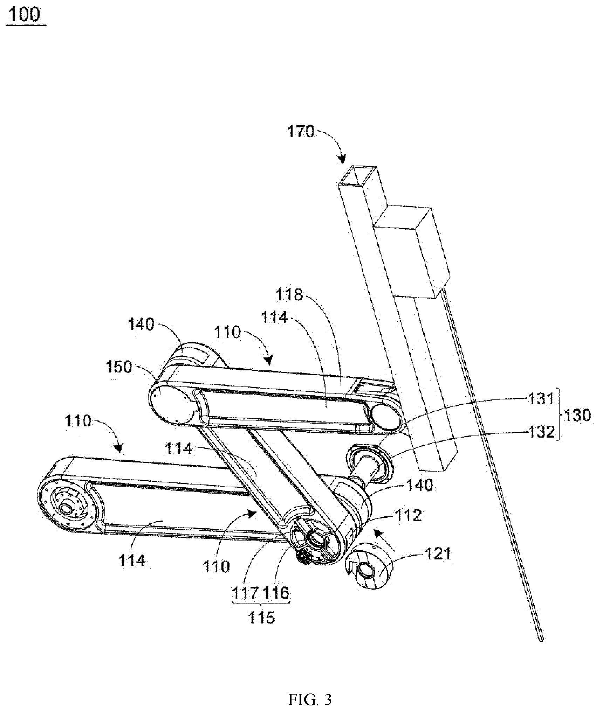

As shown in FIG. 2 and FIG. 3, the present disclosure provides a link 110, a robotic arm 100 and a robot. The robot can be a surgical robot, an industrial robot, an entertaining or teaching robot, and the like. In this embodiment, the description is made by taking a surgical robot as an example of the robot.

The robot may include at least one robotic arm 100 and an effector 170 disposed at an end of the robotic arm 100. The robotic arm 100 can drive the effector 170 to be inserted into a preset hole of the patient's skin and rotate to complete the corresponding operation. Since the effector 170 is a structure well-known in the art, it will not be described in detail.

The robotic arm 100 according to the present disclosure will be described in detail below with reference to FIG. 2 to FIG. 6.

The robotic arm 100 mainly includes at least one link 110. In the case that the robotic arm 100 includes at least two links 110, the at least two links 110 are connected end to end and two adjacent links 110 are pivotally connected. FIG. 2 and FIG. 3 exemplarily show three links 110 which are connected end to end. It can be understood that the number of the links 110 can be determined according to actual needs, and for example, it can be one, four or more.

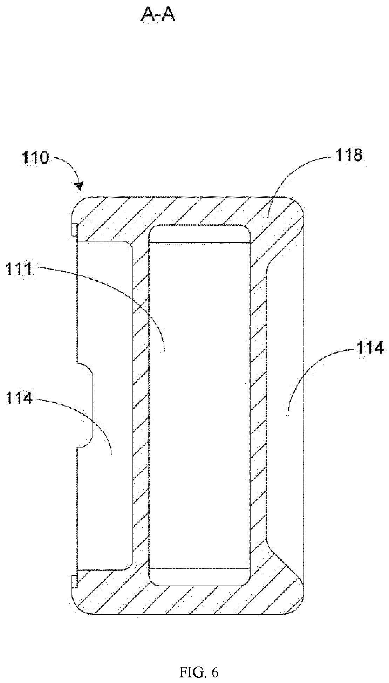

In the present embodiment, the link 110 has a length and defines an inner cavity 111 extending in a length direction L of the link 110. The inner cavity 111 extends straight from end to end of the link 110 in a direction (specifically, the length direction L of the link 110). As shown in FIG. 6, at least a part of the inner cavity 111 is seamlessly enclosed in section perpendicular to the length direction L of the link 110, so that the smooth transmission of the force flow and the continuity of the force can be ensured. In this way, the link 110 having a relative small wall thickness and a relative simple structure can meet the requirements for rigidity and torsional strength.

In the present embodiment, the cross section of the inner cavity 111 is configured in a rectangle shape, that is, the section of the inner cavity 111 perpendicular to the length direction L of the link 110 has a shape of a rectangle. Those skilled in the art can understand that the shape of the cross section of the inner cavity 111 is not limited to this embodiment and may also be a circle, an ellipse, a square, an irregular shape or any other suitable shape.

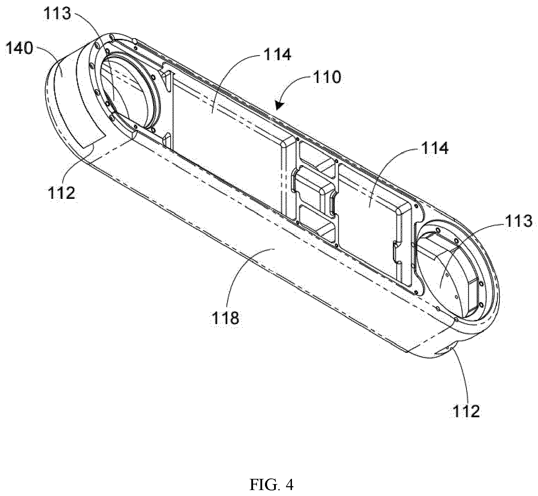

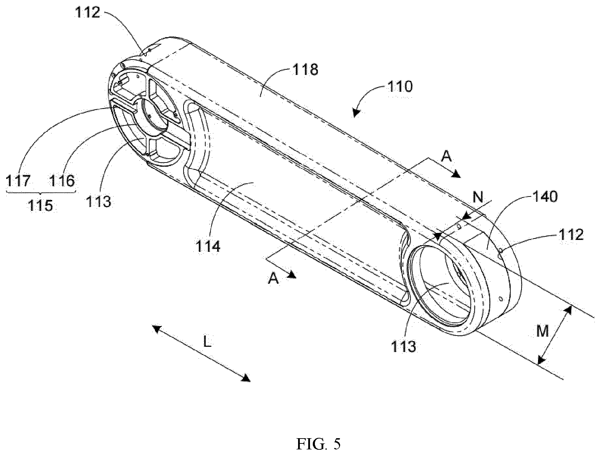

As shown in FIG. 4 and FIG. 5, the inner cavity 111 of the link 110 is in communication with two end openings 112 and two pairs of side openings 113. Preferably, each of the end openings 112 has a shape and a size which are adapted to a shape and a size of the cross section of the cavity 111 respectively. Specifically, the two end openings 112 are defined at both ends of the link 110 in the length direction L respectively and are generally configured as rectangular openings. Among the two pairs of side openings 113, one pair of side openings 113 is defined at one end of the link 110 in the length direction L and is oppositely arranged on both sides of the link 110 respectively, and the other pair of side openings 113 is defined at the other end of the link 110 in the length direction L and is oppositely arranged on both sides of the link 110 respectively. That is, the side openings 113 are disposed close to the end opening 112s, and the side openings 113 may be circular or in any other suitable shapes.

The robotic arm 100 further includes at least two flexible transmission assemblies and at least two pivot shafts 130 (see FIG. 3). In the inner cavity 111 of each link 110 is disposed at least one of the flexible transmission assemblies. In this embodiment, one flexible transmission assembly is disposed in the inner cavity 111 of each link 110, and the flexible transmission assemblies disposed in the respective inner cavities 111 of two adjacent links 110 are connected by a respective one of the pivot shafts 130. In this embodiment, the robotic arm 100 includes three flexible transmission assemblies as the robotic arm 100 includes three links 110 and each of which is provided with one flexible transmission assembly disposed in the respective inner cavity 111. It can be understood that the number of flexible transmission assemblies can be determined according to actual needs.

Specifically, the flexible transmission assembly includes a pair of pulleys 121 (see FIG. 3) and a flexible member (not shown) tensioned on the pair of pulleys 121. The pair of pulleys 121 are respectively disposed at the side openings 113 located at both ends of the inner cavity 111 of the link 110 and can enter into the inner cavity 111 through the end openings 112. In this embodiment, the flexible transmission assembly is a strap transmission assembly, the pulley 121 is a strap pulley, and the flexible member is a steel strap. In an embodiment not shown, the flexible transmission assembly may be a cable transmission assembly, and the flexible member may be a flexible cable.

Preferably, as shown in FIG. 3, the end opening 112 has a length M (see FIG. 5) which is greater than an outer diameter of the pulley 121 and the end opening 112 has a width N (see FIG. 5) which is greater than a thickness of the pulley 121, which facilitates the mounting of the pulley 121 into the inner cavity 111 through the end opening 112. The respective one of the pivot shafts 130 is connected with the pulleys 121 in the respective inner cavities 111 of the two adjacent links 110 through the side openings 113. Specifically, the pulleys 121 in the respective inner cavities 111 of the two adjacent links 110 are sleeved on the same pivot shaft 130.

As shown in FIG. 3, the pivot shaft 130 includes an end cover portion 131 and a pivot portion 132 connected with the end cover portion 131. The end cover portion 131 has a shape of a substantially disk. The pivot portion 132 is disposed at the center of the end cover portion 131 and has a shape of a cylinder extending in a direction perpendicular to the end cover portion 131. The end cover portion 131 is disposed at the side opening 113 of one of two adjacent links 110 and is connected with the link 110, and specifically, the end cover portion 131 is disposed at the side opening 113 of the link 110 which is defined on a surface facing away from the adjacent link 110. The pulleys 121 in the inner cavities 111 of the two adjacent links 110 are sleeved on the pivot portion 132 of the same pivot shaft 130.

As shown in FIG. 3 and FIG. 5, the link 110 includes a main body 118 and a supporting portion 115 integrally formed with the main body 118. The inner cavity 111 is defined by the main body 118, and the supporting portion 115 is configured to support and/or limit the position of the flexible transmission assemblies. For example, the supporting portion 115 can support and limit the position of the pulleys 121 of the flexible transmission assembly in an axial direction.

Specifically, the supporting portion 115 is disposed in the side opening 113. The supporting portion 115 may be disposed in at least one of each pair of side openings 113. The supporting portion 115 includes a first supporting rib 116 extending in a circumferential direction and a second supporting rib 117 connected with the first supporting rib 116. The second supporting rib 117 extends outward from the first supporting rib 116 in a radial direction of the first supporting rib 116. The pivot portion 132 of the pivot shaft 130 passes through an inner hole defined by the first supporting rib 116, and the first supporting rib 116 can support and limit the position of the pivot portion 132.

As shown in FIG. 2 and FIG. 3, the robotic arm 100 may further include an end cover 140 for each of the end openings 112 and a side cover 150 for each of the side openings 113. The end cover 140 has a shape and a size which correspond to a shape and a size of the end opening 112 respectively, so as to be able to completely cover the end opening 112. The side cover 150 is disposed at a respective one of the side openings 113 and connected with the link 110. Specifically, the side cover 150 may be disposed at the side opening 113 of the link 110 which is defined on a surface facing away from adjacent link 110 connected with the end cover portion 131. The end openings 112 and the side openings 113 facilitate the operation and maintenance of the transmission member such as the pulleys 121.

The link 110 according to the present embodiment is one-piece. Specifically, the link 110 may be integrally formed (for example, formed by machining such as wire cutting), may be formed by drawing and then machining, or may be formed by casting.

In addition, the link 110 includes at least one recess 114 at an outer surface of the link 110. These recesses 114 is configured to accommodate at least one of wires, circuit boards, sensors, connecting flanges and other mechanical and electrical parts. FIG. 6 shows two recesses 114 which are located respectively on two opposing sides of the link 110 and at least one of which is configured to receive wires. The link 110 may further include a cover plate (not shown) for covering each recess 114, and the cover plate is connected to the link 110 and can cover the wires.

The link 110 of the present disclosure can be used not only in the above-mentioned flexible transmission system, but also in other types of belt transmission system or wire transmission system, etc., where there is a strong demand for the miniaturization of the cavity and high rigidity.

Unless otherwise defined, technical and scientific terms used herein have the same meaning as commonly understood by those skilled in the art of the present disclosure. The terminology used herein is for the purpose of describing a particular implementation only and is not intended to limit the present disclosure. A term such as "disposed" present herein means that one element is attached to the other element directly or by an intermediary piece. Features described herein in an embodiment may be applied to the embodiment alone or in combination with other features, unless the feature is not applicable in the other embodiment or stated otherwise.

The present disclosure has been described by the above-mentioned embodiments, but it should be understood that the above-mentioned embodiments are only for the purpose of illustration and description, and are not intended to limit the present disclosure to the scope of the described embodiments. It will be understood by those skilled in the art that various variations and modifications can be made according to the teachings of the present disclosure.

## Claims

1. A robotic arm (100), comprising:
at least two links (110) connected end to end and adjacent two of which are pivotably connected, each link (110) having a length, including a first end, an intermediate portion, and a second end sequentially arranged in a length direction (L) of the link (110), and defining an inner cavity (111) extending in the length direction (L) of the link (110); wherein,
the link (110) is one-piece, and a part of the inner cavity (111) in the intermediate portion of the link (110) is seamlessly enclosed in cross section perpendicular to the length direction (L) of the link (110);
the link (110) comprises at least one recess (114) at an outer surface of the intermediate portion of the link (110) and a wall that defines the inner cavity (111), the at least one recess (114) is formed by recessing from an outer surface of the wall in a thickness direction of the wall and separated from the inner cavity (111), the inner cavity (111) is configured to accommodate at least one flexible transmission assembly, and the at least one recess is configured to accommodate a mechanical or electrical part;
the inner cavity (111) is further in communication with two pairs of side openings (113), wherein a pair of side openings (113) is defined at the first end of the link (110) in the length direction (L) and is oppositely arranged on both sides of the link (110) respectively, and the other pair of side openings (113) is defined at the second end of the link (110) in the length direction (L) and is oppositely arranged on both sides of the link (110) respectively; and
each link (110) comprises a main body (118), and the inner cavity (111) is defined by the main body (118); and
at least two flexible transmission assemblies and at least two pivot shafts (130), wherein in the inner cavity (111) of each of the links (110) is disposed at least one of the flexible transmission assemblies, and the flexible transmission assemblies disposed in the respective inner cavities of the adjacent two of the links (110) are connected by a respective one of the pivot shafts (130);
wherein each pivot shaft (130) comprises an end cover portion (131) and a pivot portion (132) connected with the end cover portion (131), the end cover portion (131) is disposed at the side opening (113) of one of two adjacent links (110) and is connected with the link (110), and particularly, the end cover portion (131) is disposed at the side opening (113) of the link (110) which is defined on a surface facing away from the adjacent link (110);
**characterized in that**,
the at least one recess (114) is disposed on a side of the link (110) where the side opening (113) is located; and
each link (110) further comprises a supporting portion (115) integrally formed with the main body (118), wherein the supporting portion (115) is configured to support a flexible transmission assembly and/or limit a position of the flexible transmission assembly, and the supporting portion (115) is disposed in the side opening (113) of the other link of two adjacent links (110) which is defined on a surface of the other link facing away from the one link (110);
wherein the supporting portion (115) comprises a first supporting rib (116) extending in a circumferential direction and a second supporting rib (117) connected with the first supporting rib (116) and extending outward from the first supporting rib (116) in a radial direction of the first supporting rib (116); and the pivot portion (132) of the pivot shaft (130) passes through an inner hole defined by the first supporting rib (116), and the first supporting rib (116) supports the pivot portion (132) and limits the position of the pivot portion (132).

2. The robotic arm (100) according to claim 1, wherein the robotic arm (100) comprises one of:
the link (110) further comprises a cover plate for covering the recess (114), the cover plate being connected to the link (110) and configured to cover a wire;
the inner cavity (111) is in communication with two end openings (112) defined at the first and second ends of the link (110) in the length direction (L) respectively;
the inner cavity (111) is defined by a peripheral wall, a portion of the peripheral wall comprising two opposing surfaces, one of which faces toward the inner cavity (111) and the other of which faces toward the recess; or
the at least one recess (114) is defined by a bottom wall and two opposing side walls, an opening of the at least one recess (114) is disposed facing the bottom wall which is a portion of the wall of the inner cavity (111).

3. The robotic arm (100) according to claim 1, wherein in the inner cavity (111) of each of the links (110) is disposed one flexible transmission assembly which comprises a pair of pulleys (121) and a flexible member tensioned on the pair of pulleys (121), the pair of pulleys (121) are respectively disposed at the side openings (113) at both ends of the inner cavity (111) and enter into the inner cavity (111) through the end openings (112), and the respective one of the pivot shafts (130) is connected with the pulleys (121) in the respective inner cavities of the adjacent two of the links (110) through the side openings (113).

4. The robotic arm (100) according to claim 1 or 2, further comprising an end cover (140) for each of the end openings (112) and a side cover (150) for each of the side openings (113), and the side cover (150) is disposed at a respective one of the side openings (113) and connected with the link (110).

5. The robotic arm (100) according to any one of claims 1 to 4, wherein the inner cavity (111) extends straight from end to end of the link (110).

6. A robot, comprising at least one robotic arm (100) according to any one of claims 1 to 5.

## Patentansprüche

1. Roboterarm (100), umfassend:
mindestens zwei Verbindungsglieder (110), die Ende an Ende verbunden sind und von denen zwei benachbarte schwenkbar verbunden sind, wobei jedes Verbindungsglied (110) eine Länge aufweist, einschließlich eines ersten Endes, eines Zwischenabschnitts und eines zweiten Endes, die in Längsrichtung (L) des Verbindungsglieds (110) aufeinanderfolgend angeordnet sind, und einen inneren Hohlraum (111) definiert, der sich in Längsrichtung (L) des Verbindungsglieds (110) erstreckt; wobei
das Verbindungsglieds (110) einteilig ist und ein Teil des inneren Hohlraums (111) im Zwischenabschnitt des Verbindungsglieds (110) im Querschnitt senkrecht zur Längsrichtung (L) des Verbindungsglieds (110) nahtlos umschlossen ist;
das Verbindungsglied (110) mindestens eine Aussparung (114) an einer Außenfläche des Zwischenabschnitts des Verbindungsglieds (110) und eine Wand umfasst, die den inneren Hohlraum (111) definiert, wobei die mindestens eine Aussparung (114) durch Auskehlen von einer Außenfläche der Wand in einer Dickenrichtung der Wand gebildet ist und von dem inneren Hohlraum (111) getrennt ist, der Innenhohlraum (111) ist so konfiguriert, dass er mindestens eine flexible Übertragungsbaugruppe aufnehmen kann, und die mindestens eine Aussparung ist so konfiguriert, dass sie ein mechanisches oder elektrisches Teil aufnehmen kann;
der Innenhohlraum (111) steht ferner mit zwei Paaren von Seitenöffnungen (113) in Verbindung, wobei ein Paar von Seitenöffnungen (113) am ersten Ende des Verbindungsglieds (110) in Längsrichtung (L) definiert ist und jeweils gegenüberliegend auf beiden Seiten des Verbindungsglieds (110) angeordnet ist, und das andere Paar von Seitenöffnungen (113) am zweiten Ende des Verbindungsglieds (110) in Längsrichtung (L) definiert ist und jeweils gegenüberliegend auf beiden Seiten des Verbindungsglieds (110) angeordnet ist; und
jedes Verbindungsglied (110) einen Hauptkörper (118) umfasst und der Innenhohlraum (111) durch den Hauptkörper (118) definiert ist; und
mindestens zwei flexible Übertragungsbaugruppen und mindestens zwei Schwenkachsen (130), wobei in dem inneren Hohlraum (111) jedes der Verbindungsglieder (110) mindestens eine der flexiblen Übertragungsbaugruppen angeordnet ist und die in den jeweiligen inneren Hohlräumen der benachbarten beiden Verbindungsglieder (110) angeordneten flexiblen Übertragungsbaugruppen durch eine jeweilige der Schwenkachsen (130) verbunden sind;
wobei jede Schwenkachsen (130) einen Endabdeckungsabschnitt (131) und einen mit dem Endabdeckungsabschnitt (131) verbundenen Schwenkabschnitt (132) umfasst, wobei der Endabdeckungsabschnitt (131) an der Seitenöffnung (113) eines von zwei benachbarten Verbindungsglieder (110) angeordnet und mit dem Verbindungsglied(110) verbunden ist, und insbesondere der Endabdeckungsabschnitt (131) an der Seitenöffnung (113) des Verbindungsglieds(110) angeordnet ist, die auf einer Fläche definiert ist, die von dem benachbarten Verbindungsglied(110) weg zeigt;
**dadurch gekennzeichnet, dass**
die mindestens eine Aussparung (114) an einer Seite des Verbindungsglieds (110) angeordnet ist, an der sich die Seitenöffnung (113) befindet; und
jedes Verbindungsglieds (110) ferner einen Stützabschnitt (115) umfasst, der integral mit dem Hauptkörper (118) ausgebildet ist, wobei der Stützabschnitt (115) so konfiguriert ist, dass er eine flexible Übertragungsbaugruppe stützt und/oder eine Position der flexiblen Übertragungsbaugruppe begrenzt, und der Stützabschnitt (115) in der Seitenöffnung (113) des anderen Glieds von zwei benachbarten Verbindungsglied (110) angeordnet ist, die auf einer Oberfläche des anderen Glieds definiert ist, die von dem einen Verbindungsglied (110) weg zeigt;
wobei der Stützabschnitt (115) eine erste Stützrippe (116), die sich in Umfangsrichtung erstreckt, und eine zweite Stützrippe (117) umfasst, die mit der ersten Stützrippe (116) verbunden ist und sich von der ersten Stützrippe (116) in einer Radialrichtung der ersten Stützrippe (116) nach außen erstreckt; und der Schwenkabschnitt (132) der Schwenkachsen (130) durch ein durch die erste Stützrippe (116) definiertes Innenloch verläuft und die erste Stützrippe (116) den Schwenkabschnitt (132) stützt und die Position des Schwenkabschnitts (132) begrenzt.

2. Roboterarm (100) nach Anspruch 1, wobei der Roboterarm (100) eines der folgenden Merkmale aufweist:
das Verbindungsglied (110) umfasst ferner eine Abdeckplatte zum Abdecken der Aussparung (114), wobei die Abdeckplatte mit dem Verbindungsglied (110) verbunden und so konfiguriert ist, dass sie einen Draht abdeckt;
der Innenhohlraum (111) mit zwei Endöffnungen (112) in Verbindung steht, die am ersten bzw. zweiten Ende des Verbindungsglieds (110) in Längsrichtung (L) definiert sind;
der Innenhohlraum (111) durch eine Umfangswand definiert ist, wobei ein Abschnitt der Umfangswand zwei gegenüberliegende Flächen umfasst, von denen eine zum Innenhohlraum (111) und die andere zur Aussparung hin ausgerichtet ist; oder
die mindestens eine Aussparung (114) durch eine Bodenwand und zwei gegenüberliegende Seitenwände definiert ist, wobei eine Öffnung der mindestens einen Aussparung (114) der Bodenwand zugewandt angeordnet ist, die ein Teil der Wand des inneren Hohlraums (111) ist.

3. Roboterarm (100) nach Anspruch 1, wobei in dem inneren Hohlraum (111) jedes der Verbindungsglieder (110) eine flexible Übertragungsbaugruppe angeordnet ist, die ein Paar Riemenscheiben (121) und ein auf dem Paar Riemenscheiben (121) gespanntes flexibles Element umfasst, wobei das Paar Riemenscheiben (121) jeweils an den seitlichen Öffnungen (113) an beiden Enden des Innenhohlraums (111) angeordnet sind und durch die Endöffnungen (112) in den Innenhohlraum (111) eintreten, und die jeweilige Schwenkwelle (130) mit den Riemenscheiben (121) in den jeweiligen Innenhohlräumen der beiden benachbarten Verbindungsglieder (110) durch die seitlichen Öffnungen (113) verbunden ist.

4. Roboterarm (100) nach Anspruch 1 oder 2, der ferner eine Endabdeckung (140) für jede der Endöffnungen (112) und eine Seitenabdeckung (150) für jede der Seitenöffnungen (113) umfasst, wobei die Seitenabdeckung (150) an einer jeweiligen Seitenöffnung (113) angeordnet und mit dem Verbindungsglied (110) verbunden ist.

5. Roboterarm (100) nach einem der Ansprüche 1 bis 4, wobei sich der Innenhohlraum (111) gerade von einem Ende des Verbindungsglieds (110) zum anderen erstreckt.

6. Roboter, der mindestens einen Roboterarm (100) nach einem der Ansprüche 1 bis 5 umfasst.

## Revendications

1. Bras robotisé (100), comprenant :
au moins deux liaisons (110) reliés bout à bout et dont deux adjacents sont reliés de manière pivotante, chaque liaison (110) ayant une longueur, comprenant une première extrémité, une partie intermédiaire et une seconde extrémité disposées séquentiellement dans une direction longitudinale (L) de la liaison (110), et définissant une cavité interne (111) s'étendant dans la direction longitudinale (L) de la liaison (110) ; dans lequel
la liaison (110) est monobloc, et une partie de la cavité interne (111) dans la partie intermédiaire de la liaison (110) est enfermée de manière homogène dans une section transversale perpendiculaire à la direction longitudinale (L) de la liaison (110) ;
la liaison (110) comprend au moins un évidement (114) au niveau d'une surface extérieure de la partie intermédiaire de la liaison (110) et une paroi qui définit la cavité interne (111), le au moins un évidement (114) est formé par évidement à partir d'une surface extérieure de la paroi dans une direction d'épaisseur de la paroi et séparé de la cavité interne (111), la cavité interne (111) est configurée pour loger au moins un ensemble de transmission flexible, et le au moins un évidement est configuré pour loger une pièce mécanique ou électrique ;
la cavité interne (111) est en outre en communication avec deux paires d'ouvertures latérales (113), dans lesquelles une paire d'ouvertures latérales (113) est définie à la première extrémité de la liaison (110) dans la direction longitudinale (L) et est disposée de manière opposée respectivement des deux côtés de la liaison (110), et l'autre paire d'ouvertures latérales (113) est définie à la deuxième extrémité de la liaison (110) dans la direction longitudinale (L) et est disposée de manière opposée respectivement des deux côtés de la liaison (110) ; et
chaque liaison (110) comprend un corps principal (118), et la cavité interne (111) est définie par le corps principal (118) ; et
au moins deux ensembles de transmission flexibles et au moins deux axes de pivotement (130), dans lesquels au moins un des ensembles de transmission flexibles est disposé dans la cavité interne (111) de chacune des liaisons (110), et les ensembles de transmission flexibles disposés dans les cavités internes respectives des deux liaisons adjacentes (110) sont reliés par un des arbres de pivotement respectifs (130) ;
dans lequel chaque axe de pivotement (130) comprend une partie de couvercle d'extrémité (131) et une partie de pivotement (132) reliée à la partie de couvercle d'extrémité (131), la partie de couvercle d'extrémité (131) est disposée au niveau de l'ouverture latérale (113) de l'une des deux liaisons adjacentes (110) et est reliée à la liaison (110), et en particulier, la partie de couvercle d'extrémité (131) est disposée au niveau de l'ouverture latérale (113) de la liaison (110) qui est définie sur une surface opposée à la liaison adjacente (110) ;
**caractérisé en ce que**
le au moins un évidement (114) est disposé sur un côté de la liaison (110) où se trouve l'ouverture latérale (113) ; et
chaque liaison (110) comprend en outre une partie de support (115) formée d'un seul tenant avec le corps principal (118), dans lequel la partie de support (115) est configurée pour supporter un ensemble de transmission flexible et/ou limiter une position de l'ensemble de transmission flexible, et la partie de support (115) est disposée dans l'ouverture latérale (113) de l'autre liaison de deux liaisons adjacentes (110) qui est définie sur une surface de l'autre liaison opposée à la première liaison (110) ;
dans lequel la partie de support (115) comprend une première nervure de support (116) s'étendant dans une direction circonférentielle et une deuxième nervure de support (117) reliée à la première nervure de support (116) et s'étendant vers l'extérieur à partir de la première nervure de support (116) dans une direction radiale de la première nervure de support (116) ; et la partie pivotante (132) de l'arbre de pivotement (130) passe à travers un trou intérieur défini par la première nervure de support (116), et la première nervure de support (116) supporte la partie pivotante (132) et limite la position de la partie pivotante (132).

2. Bras robotique (100) selon la revendication 1, dans lequel le bras robotique (100) comprend l'un des éléments suivants :
la liaison (110) comprend en outre une plaque de recouvrement pour recouvrir l'évidement (114), la plaque de recouvrement étant reliée à la liaison (110) et configurée pour recouvrir un fil;
la cavité interne (111) est en communication avec deux ouvertures d'extrémité (112) définies respectivement aux première et deuxième extrémités de la liaison (110) dans la direction longitudinale (L) ;
la cavité interne (111) est définie par une paroi périphérique, une partie de la paroi périphérique comprenant deux surfaces opposées, dont l'une fait face à la cavité interne (111) et l'autre fait face à l'évidement ; ou
le au moins un évidement (114) est défini par une paroi inférieure et deux parois latérales opposées, une ouverture du au moins un évidement (114) est disposée face à la paroi inférieure qui est une partie de la paroi de la cavité interne (111).

3. Bras robotisé (100) selon la revendication 1, dans lequel, dans la cavité interne (111) de chacune des liaisons (110), est disposé un ensemble de transmission flexible qui comprend une paire de poulies (121) et un élément flexible tendu sur la paire de poulies (121), la paire de poulies (121) étant respectivement disposée au niveau des ouvertures latérales (113) aux deux extrémités de la cavité interne (111) et pénètrent dans la cavité interne (111) par les ouvertures d'extrémité (112), et chacun des axes de pivotement (130) est relié aux poulies (121) dans les cavités internes respectives des deux liaisons adjacentes (110) par les ouvertures latérales (113).

4. Bras robotisé (100) selon la revendication 1 ou 2, comprenant en outre un couvercle d'extrémité (140) pour chacune des ouvertures d'extrémité (112) et un couvercle latéral (150) pour chacune des ouvertures latérales (113), et le couvercle latéral (150) est disposé au niveau de l'une respective des ouvertures latérales (113) et relié à la liaison (110).

5. Bras robotisé (100) selon l'une quelconque des revendications 1 à 4, dans lequel la cavité interne (111) s'étend en ligne droite d'une extrémité à l'autre de la liaison (110).

6. Robot, comprenant au moins un bras robotisé (100) selon l'une quelconque des revendications 1 à 5.
